# EUROPÄISCHE PATENTSCHRIFT

(11) **EP 0 630 928 B1**
(45) Veröffentlichungstag und Bekanntmachung des Hinweises auf die Patenterteilung: **22.10.1997**
(21) Anmeldenummer: 94109017.7
(22) Anmeldetag: 13.06.1994
(51) Int. Cl.: C08G 18/79, C08G 18/08

(54) **Verfahren zur Herstellung von Isocyanurat- und/oder Uretdiongruppen enthaltenden Polyisocyanaten mit reduzierter Farbzahl und verbesserter Lagerstabilität sowie nach diesem Verfahren hergestellte Produkte**
Process for the preparation of isocyanurate and/or urethdione groups containing polyisocyanates with a reduced colour number and an improved storage stability, and the products prepared in this way
Procédé pour la préparation de polyisolyanates contenant des groupes d'isolyanurate et/ou d'uréthdione ayant un indice de couleur réduit et une stabilité au stockage améliorée ainsi que les produits ainsi préparés

(30) Priorität: 23.06.1993 DE 4320821
(43) Veröffentlichungstag der Anmeldung: 28.12.1994
(73) Patentinhaber: BASF Aktiengesellschaft, 67063 Ludwigshafen (DE)
(72) Erfinder: Bruchmann, Bernd, Dr., D-67069 Ludwigshafen (DE); Wolff, Stefan, Dr., D-67117 Limburgerhof (DE); Stiefenhoefer, Konrad, D-67280 Ebertsheim (DE)

(56) Entgegenhaltungen:
- EP-A- 0 377 177
- EP-A- 0 481 318

## Beschreibung

Gegenstand der Erfindung ist ein Verfahren zur Herstellung von Isocyanurat- und Uretdiongruppen enthaltenden Polyisocyanatmischungen mit reduzierter Farbzahl durch katalytische Oligomerisierung von aliphatischen und/oder cycloaliphatischen Diisocyanaten und anschließende Entfernung der nicht umgesetzten Diisocyanate. Gegenstand der Erfindung sind weiterhin die nach diesem Verfahren hergestellten Produkte.

Für hochwertige Ein- und Zweikomponenten-Polyurethanlacke mit hoher Licht- und Wetterbeständigkeit werden als Isocyanatkomponente insbesondere Isocyanurat- und Uretdiongruppen enthaltende Polyisocyanatmischungen eingesetzt.

Die Herstellung dieser Produkte erfolgt vorzugsweise durch katalytische Oligomerisierung von aliphatischen und/oder cycloaliphatischen Diisocyanaten, z.B. 1-Isocyanato-3,3,5-trimethyl-5-iso-cyanatomethylcyclohexan (IPDI) oder 1,6-Diisocyanatohexan (HDI).

Als Katalysatoren können beispielsweise Hydroxide oder organische Salze von schwachen Säuren mit Tetraalkylammonium-Gruppen, Hydroxide oder organische Salze von schwachen Säuren mit Hydroxyalkylammonium-Gruppen, Alkalimetallsalze oder Zinn-, Zink- bzw. Bleisalze von Alkylcarbonsäuren eingesetzt werden.

Hierbei läßt man die aliphatischen und/oder cycloaliphatischen Diisocyanate in Gegenwart des Katalysators, gegebenenfalls unter Verwendung von Lösungsmitteln und/oder Hilfsstoffen, bis zum Erreichen des gewünschten Umsatzes reagieren. Danach wird die Reaktion durch Desaktivierung des Katalysators abgebrochen und das überschüssige monomere Diisocyanat abdestilliert. Je nach dem verwendeten Katalysatortyp und der Reaktionstemperatur erhält man Polyisocyanate mit unterschiedlichen Anteilen an Isocyanurat- bzw. Uretdiongruppen.

Die so hergestellten Produkte sind meist klare, aber in Abhängigkeit vom Katalysatortyp, der Diisocyanatqualität, der Reaktionstemperatur und der Reaktionsfahrweise mehr oder weniger stark gelbgefärbte Produkte.

Für die Herstellung hochwertiger Polyurethanlacke sind jedoch Produkte mit einer möglichst niedrigen Farbzahl erwünscht. Im Stand der Technik sind eine Reihe von Verfahren zur Farbzahlsenkung derartiger Produkte bekannt.

So wird in DE-A-38 06 276 vorgeschlagen, den Kohlendioxidgehalt des als Monomeres eingesetzten HDI vor der Oligomerisierung durch Entgasen unter Vakuum und anschließendes Durchblasen von Stickstoff durch das HDI auf weniger als 20 ppm zu senken sowie als Oligomerisierungskatalysator quaternäre Ammoniumhydroxide einzusetzen. Der Verfahrensschritt der Kohlendioxidentfernung ist jedoch technisch sehr aufwendig.

In der EP-A-0 339 396 wird der Einsatz von quaternären Ammoniumfluoriden als Trimerisierungskatalysator vorgeschlagen. Bei diesem Verfahren kann zwar ein höherer Kohlendioxidgehalt toleriert werden, der vorgeschlagene Katalysator muß jedoch chemisch desaktiviert werden. Die dabei resultierenden Verbindungen verbleiben im Produkt und können zu anwendungstechnischen Problemen bei der Weiterverarbeitung führen. Eine weitere Möglichkeit zur Herstellung von Isocyanuratgruppen enthaltenden Polyisocyanaten mit niedrigerer Farbzahl besteht in dem in der EP-A-0 336 205 vorgeschlagenen Zusatz von Polyesterdiolen zum Ausgangsdiisocyanat. Dadurch kann der Katalysatoreinsatz reduziert werden; die resultierenden Produkte sind jedoch immer noch relativ stark gefärbt.

In der EP-A-0 377 177 wird das aliphatische Diisocyanat in Gegenwart von Phosphinen als Katalysator oligomerisiert, nach Abbruch der Oligomerisierung wird das nicht umgesetzte Diisocyanat zum Teil abdestilliert, zum Teil durch Zusatz von Alkohol in Urethan umgewandelt. Anschließend wird das Reaktionsprodukt mit Peroxiden behandelt. Durch die Peroxidbehandlung kommt es zwar zu einer Senkung der Farbzahl des Oligomerisierungsprodukts, der Einsatz von Peroxiden ist jedoch oftmals mit Problemen verbunden. So sind Peroxide technisch schwer zu handhaben. Handhabungssicherere Peroxide werden meist in Lösung angeboten, wobei dann das häufig als Lösungsmittel verwendete Dibutylphtalat bei der Lackherstellung zu anwendungstechnischen Problemen führt.

In EP-A-481 318 wird ein Verfahren zur Oligomerisierung von aliphatischen Diisocyanaten unter Verwendung von Katalysatoren, die dreiwertigen Phosphor enthalten, beschrieben. Zur Beendigung der Umsetzung wird der Katalysator desaktiviert, indem der dreiwertige Phosphor zum fünfwertigen Phosphor oxidiert wird.

Ein wesentlicher Nachteil der Verfahren des Standes der Technik besteht weiterhin darin, daß die Lagerstabilität der so hergestellten Produkte ungenügend ist. Es kommt zu einer Verschlechterung der Farbwerte sowie insbesondere zu einem deutlichen Anstieg der Viskosität.

Aufgabe der Erfindung war es daher, ein einfaches Verfahren zur Herstellung Isocyanurat- und/oder Uretdiongruppen enthaltender Polyisocyanate mit reduzierter Farbzahl zu entwickeln, das die Nachteile des Standes der Technik vermeidet und insbesondere zu Produkten mit einer verbesserten Lagerstabilität führt.

Erfindungsgemäß wird die Aufgabe überraschenderweise gelöst durch ein Verfahren zur Herstellung von Isocyanurat- und/oder Uretdiongruppen enthaltenden Polyisocyanaten durch an sich bekannte katalytische Oligomerisierung aliphatischer und/oder cycloaliphatischer Diisocyanate, bei dem die Oligomerisierungsprodukte zur Farbaufhellung mit Peroxycarbonsäuren behandelt werden.

Gegenstand der Erfindung sind weiterhin die nach diesem Verfahren hergestellten Isocyanurat- und/oder Uretdiongruppen enthaltenden Polyisocyanate mit reduzierter Farbzahl.

In einer besonders vorteilhaften Ausführungsform der Erfindung werden bei Verwendung basischer, insbesondere aminischer merisierungskatalysatoren die Peroxycarbonsäuren bei Erreichen des gewünschten Oligomerisierungsgrades zur Desaktivierung des Katalysators zugesetzt und danach das Oligomerisierungsprodukt wie im Stand der Technik üblich durch Entmonomerisierung, zumeist unter Hochvakuum mittels Dünnschichtverdampfer, aufgearbeitet. Ein besonderer Verfahrensschritt zur Aufhellung der Oligomerisierungsprodukte ist dann nicht mehr erforderlich.

Die Oligomerisierung der aliphatischen und/oder cycloaliphatischen Diisocyanate erfolgt wie im Stand der Technik üblich.

Als Ausgangsdiisocyanate werden aliphatische und/oder cycloaliphatische Diisocyanate, z.B. 1,4-Diisocyanatohexan, 1,6-Diisocyanatohexan (HDI), 1,12-Diisocyanatododecan, 1-Isocyanato-3,3,5-trimethyl-5-isocyanatomethyl-cyclohexan(IPDI), 4,4'-Diisocyanatodicyclohexylmethan, 1,5-Diisocyanato-2,2-dimethylpentan, 1,5-Diisocyanato-2-ethyl-2-propylpentan, 1,6-Diisocyanato-2,4,4-trimethylhexan, 1,5-Diisocyanato-2-methylpentan, insbesondere HDI, eingesetzt.

Die Oligomerisierung der Diisocyanate wird vorzugsweise bei Temperaturen im Bereich von 0°C bis 100°C unter Durchleiten von Inertgas, vorzugsweise Stickstoff, durchgeführt. Bei niedrigeren Temperaturen ist die Reaktionsgeschwindigkeit zu gering, bei höheren Temperaturen laufen verstärkt Nebenreaktionen ab.

Als Katalysatoren werden Hydroxide oder organische Salze von schwachen Säuren mit Tetraalkylammonium-Gruppen, Hydroxide oder organische Salze von schwachen Säuren mit Hydroxyalkylammonium-Gruppen, Alkalimetallsalze oder Zinn-, Zink- bzw. Bleisalze von Alkylcarbonsäuren eingesetzt. Die Katalysatoren werden üblicherweise in einer Menge von 0,05 Gew.-% bis 2 Gew.-%, bezogen auf das Diisocyanat, eingesetzt.

Zur Reduzierung der Katalysatormenge ist es möglich, den Diisocyanaten vor der Oligomerisierung in an sich bekannter Weise eine geringe Menge, bis ca. 1 Gewichts-% bezogen auf das Diisocyanat, eines Diols, insbesondere eine Polyesterdiols, zuzusetzen.

Danach wird das Diisocyanat unter Rühren auf die Reaktionstemperatur gebracht und der Katalysator langsam zugegeben. Zur besseren Handhabung kann der Katalysator in einem Lösungsmittel gelöst werden. Es eignen sich hierzu beispielsweise Alkohole, insbesondere Diole, Ketone, Ether und Ester.

Nach Erreichen des gewünschten Umsetzungsgrades wird die Reaktion durch Desaktivierung des Katalysators, beispielsweise durch Zugabe eines Katalysatorgifts oder durch thermische Zersetzung des Katalysators, gestoppt. Anschließend wird die Reaktionsmischung in geeigneter, an sich bekannter Weise, etwa durch Destillation, beispielsweise mittels Dünnschichtverdampfer, von den monomeren Diisocyanaten befreit.

Eine besonders günstige und daher bevorzugte Ausführungsform der Erfindung besteht wie schon erwähnt darin, die Peroxycarbonsäuren als Neutralisierungsmittel für die eingesetzten basischen Oligomerisierungskatalysatoren einzusetzen. Dabei werden dem Reaktionsgemisch bei Erreichen des gewünschten Oligomerisierungsgrades zur Desaktivierung des Katalysators und damit zum Abbruch der Reaktion die erfindungsgemäßen Peroxycarbonsäuren in einer Menge von 10 bis 10 000 ppm, vorzugsweise 50 bis 1 000 ppm, bezogen auf die Reaktionsmischung, zugesetzt. Die Peroxycarbonsäuren können auch gemeinsam mit herkömmlichen Neutralisationsmitteln eingesetzt werden. Anschließend wird die Reaktionsmischung in geeigneter, an sich bekannter Weise, etwa durch Destillation, beispielsweise mittels Dünnschichtverdampfer, von den monomeren Diisocyanaten befreit.

Möglich im Sinne der Erfindung ist es jedoch auch, bei Erreichen des gewünschten Oligomerisierungsgrades den Katalysator in anderer geeigneter Weise, z.B. mittels Katalysatorgiften oder durch thermische Zersetzung, zu desaktivieren. Anschließend wird in der beschriebenen Weise die Reaktionsmischung von den monomeren Diisocyanaten befreit. Zu der so aufgearbeiteten Lösung werden die erfindungsgemäßen Peroxycarbonsäuren in einer Menge von 10 bis 10 000 ppm, vorzugsweise 50 bis 1 000 ppm, bezogen auf die Diisocyanatmenge, zugesetzt.

Als Peroxycarbonsäuren können aromatische, aliphatische, cycloaliphatische, gegebenenfalls auch substituierte Peroxycarbonsäuren eingesetzt werden. Möglich ist auch der Einsatz saurer Salze dieser Peroxycarbonsäuren.

Als Peroxycarbonsäuren seien beispielhaft genannt Peressigsäure, Peroxymaleinsäure, tert.-Butylperoxymaleinsäure, Perbenzoesäure, p-Nitroperbenzoesäure, Peroxyphthalsäure, insbesondere 3-Chlorperbenzoesäure. Als saure Salze der Peroxycarbonsäuren kommen insbesondere Ammonium- oder Magnesiumsalze zum Einsatz.

Das erfindungsgemäße Verfahren zur Herstellung von Isocyanurat- und/oder Uretdiongruppen enthaltenden Polyisocyanaten führt zu Produkten mit niedrigen Farbzahlen. Die Farbzahlen liegen unter 50 HAZEN, meist jedoch unter 30 HAZEN. Besonders vorteilhaft bei den erfindungsgemäß hergestellten Produkten ist jedoch ihre sehr gute Lagerstabilität. Auch nach einer Lagerzeit von 6 Monaten unter Stickstoffatmosphäre kam es zu keiner Veränderung der Farbe, und auch der Viskositätsanstieg dieser Produkte war deutlich geringer als bei solchen, die nach dem Verfahren des Standes der Technik hergestellt worden waren.

Ein zusätzlicher vorteilhafter Effekt trat bei der Neutralisation des Oligomerisierungskatalysators mit Peroxycarbonsäuren auf. Hier zeigte das nach der Oligomerisierung der aliphatischen und/oder cycloaliphatischen Diisocyanate abdestillierte monomere Diisocyanat nicht die sonst bei den entsprechenden Produkten der Verfahren nach dem Stand der Technik häufig auftretenden Nachteile.

Während bei der Neutralisierung des Oligomerisierungskatalysators mit den bei den Verfahren des Standes der Technik zumeist verwendeten Phosphorsäurealkylestern die Reaktivität der zurückgeführten Diisocyanat-Monomeren deutlich geringer war als bei frischem Diisocyanat und daher mit erhöhtem Katalysatoreinsatz gearbeitet werden muß, zeigte das bei der Neutralisation des Oligomerisierungskatalysators mit Peroxycarbonsäuren zurückgeführte monomere Diisocyanat kein derartiges Verhalten und konnte bei den üblichen Verfahrensbedingungen und mit üblichem Katalysatoreinsatz problemlos oligomerisiert werden.

Bei der ebenfalls aus dem Stand der Technik bekannten thermischen Zersetzung des Oligomerisierungsprodukts weist das zurückgeführte monomere Diisocyanat zwar meist keine Reaktivitätsunterschiede zu frischem Diisocyanat auf, es kommt jedoch häufig zum Gelieren dieser Produkte. Auch ein derartiges Verhalten konnte bei den nach dem erfindungsgemäßen Verfahren gewonnenen monomeren Diisocyanaten nicht beobachtet werden.

Die Verwendung der erfindungsgemäßen Produkte erfolgt zumeist als Isocyanatkomponente in Polyurethan-Ein- und Zweikomponentenlacken.

Die Erfindung soll an nachfolgenden Beispielen näher erläutert werden.

### Beispiel 1 (Vergleich)

500 g 1,6-Diisocyanatohexan (HDI) wurden unter Stickstoffbedeckung auf 80°C aufgeheizt und unter Rühren mit 400 ppm N,N,N-Trimethyl-N-(2-hydroxypropyl)ammonium-2-ethylhexanoat, gelöst in 2 ml Methylethylketon (MEK), versetzt.

Bei einem NCO-Gehalt der Reaktionsmischung von 43 Gew.-% wurden 2 Mol-Äquivalent, bezogen auf die Katalysatormenge, Dibutylphosphat zugegeben und 1 h bei 80°C weitergerührt. Anschließend wurde nicht umgesetztes HDI im Hochvakuum über einen Dünnschichtverdampfer abgezogen.

Die Farbzahl des oligomerisierten HDI betrug 78 HAZEN. Anschließend wurden 250 g des abgezogenen monomeren HDI unter Rühren mit 400 ppm N,N,N-Trimethyl-N-(2-hydroxypropyl)ammonium-2-ethylhexanoat versetzt. Eine Abnahme des NCO-Gehaltes konnte nicht beobachtet werden.

### Beispiel 2 (Vergleich)

Es wurde verfahren wie in Beispiel 1, nur daß zur Desaktivierung des Katalysators die Reaktionsmischung für 15 Minuten auf 110°C erhitzt wurde.

Das abgezogene monomere HDI war in der Reaktivität normal, es gelierte jedoch beim Stehen in einem geschlossenen Gefäß unter Stickstoff bei 5°C.

### Beispiel 3

Es wurde verfahren wie in Beispiel 1, nur daß zur Neutralisation des Katalysators 2 Mol-Äquivalente, bezogen auf den Katalysator, 3-Chlorperbenzoesäure eingesetzt wurden.

Die Farbzahl des Oligomerisierungsproduktes betrug 25 HAZEN. Von dem abgezogenen monomeren HDI wurden 200 g bei 80°C mit 400 ppm N,N,N-Trimethyl-N-(2-hydroxypropyl)ammonium-2-ethylhexanoat, gelöst in 2 ml MEK, versetzt.

Die NCO-Abnahme verlief wie bei frischem monomeren HDI. Bei Erreichen eines NCO-Gehaltes von 43 Gew.-%, bezogen auf die Reaktionsmischung, wurde der Katalysator durch Zugabe von 2 Mol-Äquivalenten 3-Chlorperbenzoesäure neutralisiert. Das Oligomerisierungsprodukt hatte eine Farbzahl von 20 HAZEN. 50 g des abgezogenen monomeren HDI wurden bei 5°C 4 Wochen unter Stickstoff in einem geschlossenen Gefäß gelagert. Es trat keine Gelbildung ein.

### Beispiel 4

Es wurde verfahren wie in Beispiel 1, nur daß zur Neutralisation des Katalysators 2 Mol-Äquivalente 4-Nitroperbenzoesäure eingesetzt wurden. Die Farbzahl des Oligomerisierungsproduktes betrug 27 HAZEN.

### Beispiel 5

Es wurde verfahren wie in Beispiel 1, nur daß zur Neutralisation des Katalysators 2 Mol-Äquivalente Monoperoxyphthalsäure-Magnesiumsalz eingesetzt wurden. Die Farbzahl des Oligomerisierungsproduktes betrug 50 HAZEN.

### Beispiel 6 (Vergleich)

Uretdion- und Isocyanuratgruppen enthaltendes HDI, hergestellt nach Beispiel 2, wurde nach der Abtrennung des monomeren HDI unter Stickstoff über einen Zeitraum von 6 Monaten bei 50°C gelagert. Die Viskosität stieg in dieser Zeit von 2 460 mPas bei 25°C auf 5 870 mPas bei 25°C an. Die Farbzahl des Produktes blieb mit 30 HAZEN konstant.

### Beispiel 7

Dem in Beispiel 6 verwendeten Uretdion- und Isocyanuratgruppen enthaltenden HDI wurden nach der Abtrennung des monomeren HDI 300 ppm 3-Chlorperbenzoesäure zugesetzt. Das so behandelte Produkt wurde bei 50°C unter Stickstoff über einen Zeitraum von 6 Monaten gelagert. Die Viskosität des Produktes stieg von 2 460 mPas bei 25°C auf 2 920 mPas bei 25°C an, die Farbzahl blieb mit 10 HAZEN konstant.

Die Bestimmung der HAZEN-Farbzahl erfolgte nach DIN 53 995.

## Patentansprüche

1. Verfahren zur Herstellung von Isocyanurat- und/oder Uretdiongruppen enthaltenden Polyisocyanaten mit reduzierter Farbzahl und verbesserter Lagerstabilität durch katalytische Oligomerisierung aliphatischer und/oder cycloaliphatischer Diisocyanate, dadurch gekennzeichnet, daß als Oligomerisierungskatalysatoren Hydroxide oder organische Salze von schwachen Säuren mit Tetraalkylammoniumgruppen, Hydroxide oder organische Salze von schwachen Säuren mit Hydroxyalkylammoniumgruppen, Alkalimetallsalze oder Zinn-, Zink- oder Bleisalze von Alkylcarbonsäuren eingesetzt und die Oligomerisierungsprodukte mit Peroxycarbonsäuren behandelt werden.

2. Verfahren nach Anspruch 1, dadurch gekennzeichnet, daß das Ausgangsdiisocyanat 1,6-Diisocyanatohexan ist.

3. Verfahren nach Anspruch 1, dadurch gekennzeichnet, daß man die Peroxycarbonsäuren zur Desaktivierung des Oligomerisierungskatalysators einsetzt und das Oligomerisierungsprodukt danach wie üblich weiterbehandelt wird.

4. Verfahren nach Anspruch 1, dadurch gekennzeichnet, daß die Peroxycarbonsäuren dem Oligomerisierungsprodukt nach der Entfernung der nichtumgesetzten monomeren Diisocyanate zugesetzt werden.

5. Verfahren nach einem der Ansprüche 1 bis 4, dadurch gekennzeichnet, daß die Peroxycarbonsäuren in einer Menge von 10 bis 10 000 ppm, bezogen auf das eingesetzte Diisocyanat, eingesetzt werden.

6. Verfahren nach einem der Ansprüche 1 bis 5, dadurch gekennzeichnet, daß die Peroxycarbonsäuren in einer Menge von 50 bis 1 000 ppm, bezogen auf das eingesetzte Diisocyanat, eingesetzt werden.

7. Isocyanurat- und Uretdiongruppen enthaltende Polyisocyanate mit reduzierter Farbzahl, hergestellt nach einem der Ansprüche 1 bis 6.

8. Verwendung von Isocyanurat- und Uretdiongruppen enthaltenden Polyisocyanaten nach Anspruch 7 als Isocyanatkomponente Polyurethan-Ein- und Zweikomponentenlacken.

## Claims

1. A process for the preparation of isocyanurates and/or uretdione-containing polyisocyanates having a reduced color index and improved shelf life by catalytic oligomerization of aliphatic and/or cycloaliphatic diisocyanates, wherein the oligomerisation catalysts used are hydroxides or organic salts of weak acids having tetraalkylammonium groups, hydroxides or organic salts of weak acids having hydroxyalkylammonium groups, alkali metal salts or tin, zinc or lead salts of alkanecarboxylic acids and the oligomerization products are treated with peroxycarboxylic acids.

2. A process as claimed in claim 1, wherein the starting diisocyanate is 1,6-diisocyanatohexane.

3. A process as claimed in claim 1, wherein the peroxycarboxylic acids are used for deactivating the oligomerization catalyst, and the oligomerization product is then further treated in a conventional manner.

4. A process as claimed in claim 1, wherein the peroxycarboxylic acids are added to the oligomerization product after the removal of the unconverted monomeric diisocyanates.

5. A process as claimed in any of claims 1 to 4, wherein the peroxycarboxylic acids are used in an amount of from 10 to 10,000 ppm, based on the diisocyanate used.

6. A process as claimed in any of claims 1 to 5, wherein the peroxycarboxylic acids are used in an amount of from 50 to 1,000 ppm, based on the diisocyanate used.

7. An isocyanurate- and uretdione-containing polyisocyanate having a reduced color index, prepared as claimed in any of claims 1 to 6.

8. Use of an isocyanurate- and uretdione-containing polyisocyanate as claimed in claim 7 as an isocyanate component of polyurethane one-component and two-component finishes.

## Revendications

1. Procédé de préparation de polyisocyanates contenant des radicaux isocyanurate et/ou uretdione à indice de coloration réduit et à stabilité à la conservation améliorée, par l'oligomérisation catalytique de diisocyanates aliphatiques et/ou cycloaliphatiques, caractérisé en ce que l'on utilise, à titre de catalyseurs d'oligomérisation, des hydroxydes ou des sels organiques d'acides faibles avec des radicaux tétraalkylammonium, des hydroxydes ou des sels organiques d'acides faibles avec des radicaux hydroxyalkylammonium, des sels de métaux alcalins ou des sels d'étain, de zinc ou de plomb d'acides alkylcarboxyliques et on traite les produits de l'oligomérisation par des acides peroxycarboxyliques.

2. Procédé suivant la revendication 1, caractérisé en ce que le diisocyanate de départ est le 1,6-diisocyanatohexane.

3. Procédé suivant la revendication 1, caractérisé en ce que l'on utilise les acides peroxycarboxyliques pour la désactivation du catalyseur d'oligomérisation et on poursuit ensuite le traitement du produit de l'oligomérisation comme d'habitude.

4. Procédé suivant la revendication 1, caractérisé en ce que l'on ajoute les acides peroxycarboxyliques au produit de l'oligomérisation, après l'élimination des diisocyanates monomériques non convertis.

5. Procédé suivant l'une quelconque des revendications 1 à 4, caractérisé en ce que l'on utilise les acides peroxycarboxyliques en une proportion de 10 à 10.000 ppm, par rapport au diisocyanate mis en oeuvre.

6. Procédé suivant l'une quelconque des revendications 1 à 4, caractérisé en ce que l'on utilise les acides peroxycarboxyliques en une proportion de 50 à 1000 ppm, par rapport au diisocyanate mis en oeuvre.

7. Polyisocyanates contenant des radicaux isocyanurate et uretdione à indice de coloration réduit, préparés selon le procédé suivant l'une quelconque des revendications 1 à 6.

8. Utilisation de polyisocyanates contenant des radicaux isocyanurate et uretdione suivant la revendication 7, à titre de composant du type isocyanate de laques polyuréthanniques à un ou deux composants.
